# EUROPEAN PATENT APPLICATION

(11) **EP 2 140 871 A1**
(43) Date of publication of application: **06.01.2010**
(21) Application number: 08739562.0
(22) Date of filing: 01.04.2008
(51) Int. Cl.: A61K 31/765, A61K 9/10, A61K 47/02, A61K 47/10, A61K 47/32, A61P 27/02

(54) **SUSPENSION FOR VISUALIZATION OF TRANSPARENT TISSUE IN EYE**

(30) Priority: 06.04.2007 JP 2007100060
(71) Applicant: Senju Pharmaceutical Co., Ltd., Osaka-shi, Osaka 541-0046 (JP)
(72) Inventor: MATSUHISA, Keiichi, Kobe-shi Hyogo 651-2241 (JP)
(74) Representative: Behnisch, Werner
(86) International application number: PCT/JP2008/056449
(87) International publication number: WO 2008/126720

(57) **Abstract**

Disclosed is an ocular transparent tissue-visualizing suspension which can be used as an easy-to-handle and sufficiently safe means to enhance visibility of transparent tissues of the eye during a surgical operation on them. The ocular transparent tissue-visualizing suspension comprises, in an aqueous medium, fine particles of a biodegradable macromolecular compound and at least one salt selected from the groups consisting of salts of trivalent metals and salts of divalent metals.

## Description

### TECHNICAL FIELD

The present invention relates to an ocular transparent tissue-visualizing suspension which is designed to be infused into transparent tissues of the eye in order to enhance visibility of the transparent tissues of the eye during surgical operation.

### BACKGROUND ART

To receive light from the surrounding environment and project it on the photoreceptor cells, many portions of the eyeball are made of transparent tissues, which are the cornea, lens and vitreous body. The vitreous body, which adjoins the retina, provides scaffolds to proliferating tissues formed in the retina in many of retinal diseases including diabetic retinitis. Tissues that have proliferated into the vitreous body will form fibers there, which then will draw the retina and thereby could cause retinal detachment. Thus, such a condition, if left untreated, could eventually lead to blindness. A surgical operation, therefore, is often performed to completely remove such a vitreous body that has come to contain proliferating tissues.

In vitrectomy, it is required to remove, completely as far as possible, not only the proliferating tissues that have adhered to the retina but also the vitreous body, which provides scaffolds for the proliferation of such tissues. The operation is carried out while simultaneously infusing into the eyeball an intraocular irrigating solution for surgical use. The vitreous body, however, is a transparent tissue, whose refractive index differs very little from that of intraocular irrigating solutions. Thus, when no countermeasure is taken, the transparent tissue lacks visibility in the field seen through an operation microscope, and this makes it hard to locate the tissue, thus making complete removal of it no easy task. As a means to address this problem, a steroid suspension such as a triamcinolone preparation (Kenacort-A : registered trademark) is infused during vitrectomy into a vitreous cavity, which is a space created by sucking and removing central part of the vitreous body, in order for letting the suspension disperse and adhere to the vitreous body, thereby visualizing the vitreous body (see Non-patent Document 1). This method enhances the visibility of the vitreous body in the operative field, and thus makes the surgical operation easier, allowing complete removal of the vitreous body. However, elevation of the intraocular pressure and progression of cataract have been reported to occur as the side effects associated with application of a steroid preparation to the vitreous body (see Non-patent Documents 2 and 3). Thus, similar side effects might be induced also by a steroid suspension used for the purpose of improving visibility during vitrectomy.

On the other hand, there is reported a method to visualize proliferating membranes and epiretinal membranes which have developed in proliferative vitreoretinopathy (PVR), by staining them with an aqueous solution containing a dissolved dye, such as trypan blue (see Patent Document 1). However, improvement in visibility by the method is quite insufficient, for the dye thus employed darkens the operative field and, besides, does not serve to emphasize the vitreous body in the operative field.

Also in the case of cataract surgery, for example, in which the nucleus and cortex enclosed in the lens capsule are removed before the insertion of an intraocular lens, it is known that remaining cortex that could not be removed at and near the posterior capsule often proliferates and becomes opaque with the lapse of time after operation, thus leading to the development of postoperative cataract. Therefore, with regard to cataract surgery also, a means is needed to make complete removal of the cortex easier, by visualizing the transparent part of the cortex.

As a means to improve the situation mentioned above, the present inventor previously reported a transparent tissue-visualizing preparation, which comprises fine particles of a macromolecular compound, and is designed to be brought into contact with transparent tissues of the eye, such as the vitreous body and the lens, to enhance their visibility (Patent Document 2). However, according to the result of further studies, though this transparent tissue-visualizing preparation enables to considerably improve the visibility of transparent tissues, in actual operation, part of the transparent tissue-visualizing preparation may be washed out depending on the flow rate of the irrigating solution used during the operation, thus leading to lowered visibility. Therefore, in order to permit easier and surer removal of transparent tissues in the surgery, it is highly desirable that a new transparent tissue-visualizing preparation be available which has much higher visualizing ability.
[Patent Document 1] WO 99/058159
[Patent Document 2] WO 2005/115411
[Non-patent Document 1] Sakamoto, T., et al., Graefe's Archive for Clinical and Experimental Ophthalmology, 240: p.423 (2002).
[Non-patent Document 2] Challa, J.K. et al., Australian and New Zealand Journal of Ophthalmology, 26: p.277 (1998)
[Non-patent Document 3] Wingate, R.J. et al., Australian and New Zealand Journal of Ophthalmology, 27: p.431 (1999)

### DISCLOSURE OF INVENTION

### [Problem to be solved by the invention]

Against the above background, the objective of the present invention is to provide a means to enhance visibility of the transparent tissues of the eye, i.e., the lens, the vitreous body, and the cornea, during surgical operation on them, wherein the means achieves improved sufficient visualization, is easy to use and highly safe.

To solve the above-mentioned problem, the present inventor prepared suspensions by dispersing fine particles of pharmacologically inactive biodegradable macromolecular compounds in aqueous media containing a divalent metal ion at or above a predetermined concentration and/or a trivalent metal ion at or above a predetermined concentration, and infused each suspension into the eye to bring it to contact with the transparent tissues of the eye. As a result of the study, the present inventor found that the suspension remarkably increased the scattering intensity of visible light in the operative field and greatly augmented the visibility of the transparent tissues, compared with a suspension containing no such divalent or trivalent metal ions above those predetermined concentrations. The present invention was completed on the basis of this finding and through further studies. Thus, the present invention provides what follows.

### [Means to solve the problem]

1. An ocular transparent tissue-visualizing suspension comprising, in an aqueous medium, fine particles of a biodegradable macromolecular compound and at least one salt selected from the group consisting of salts of divalent metals and salts of trivalent metals, wherein the suspension is designed to be infused into the eye to be brought into contact with transparent tissues of the eye to enhance the visibility thereof.
2. The ocular transparent tissue-visualizing suspension according to (1) above, wherein the fine particles of a biodegradable macromolecular compound are those one g of which cannot completely be dissolved in less than 30 mL of water within 30 minutes at 20 °C.
3. The ocular transparent tissue-visualizing suspension according to (1) or (2) above, wherein the biodegradable macromolecular compound is selected from the group consisting of polylactic acid, polyglycolic acid, and copoly(lactic acid/glycolic acid).
4. The ocular transparent tissue-visualizing suspension according to one of (1) to (3) above, wherein the concentration of the salt of a trivalent metal is 0.01-1 w/v% and/or the concentration of the salt of a divalent metal is 0.1-1 w/v%.
5. The ocular transparent tissue-visualizing suspension according to one of (1) to (4) above, wherein the salt of a trivalent metal is selected from aluminum salts, and iron salts, and wherein the divalent salt is selected from magnesium salts, calcium salts, and zinc salts.
6. The ocular transparent tissue-visualizing suspension according to one of (1) to (5) above, wherein the salt of a trivalent metal is aluminum potassium sulfate.
7. The ocular transparent tissue-visualizing suspension according to one of (1) to (6) above, wherein the salt of a divalent metal is magnesium chloride or calcium chloride.
8. The ocular transparent tissue-visualizing suspension according to one of (1) to (7) above, further comprising a polyvinyl-based compound which may be polyvinylpyrrolidone or polyvinylalcohol and/or a polyol.
9. The ocular transparent tissue-visualizing suspension according to (8) above, wherein the polyvinyl-based compound is polyvinylpyrrolidone and the polyol is mannitol.
10. The ocular transparent tissue-visualizing suspension according to one of (1) to (9) above, wherein content of the fine particles of the biodegradable macromolecular compound is 0.005-10 w/v%.
11. A method for preparation of an ocular transparent tissue-visualizing suspension comprising the steps of
   providing a solid preparation comprising fine particles of a biodegradable macromolecular compound,
   providing an aqueous solution containing at least one salt selected from the group consisting of salts of trivalent metals and salts of divalent metals, wherein the concentration of the salt of a trivalent metal is not less than 0.01 w/v%, and/or wherein the concentration of the salt of a divalent metal is not less than 0.1 w/v%, and
   suspending the fine particles in the aqueous solution.
12. The method for preparation of an ocular transparent tissue-visualizing suspension according to (11) above, wherein the fine particles of a biodegradable macromolecular compound are those one g of which cannot completely be dissolved in less than 30 mL of water within 30 minutes at 20 °C.
13. The method for preparation of an ocular transparent tissue-visualizing suspension according to (11) or (12) above, wherein the solid preparation and/or the aqueous solution further comprises at least one compound selected from the group consisting of polyvinyl-based compounds and polyols.
14. A method for preparation of an ocular transparent tissue-visualizing suspension comprising the steps of
   providing a solid preparation comprising fine particles of a biodegradable macromolecular compound and at least one salt selected from the group consisting of salts of trivalent metals and salts of divalent metals, and
   forming a suspension by mixing the solid preparation with an aqueous medium so that the concentration of the salt of a trivalent metal is not less than 0.01 w/v% and/or the concentration of the salt of a divalent metal is not less than 0.1 w/v%.
15. The method for preparation of an ocular transparent tissue-visualizing suspension according to (14) above, wherein the fine particles of a biodegradable macromolecular compound are those one g of which cannot completely be dissolved in less than 30 mL of water within 30 minutes at 20 °C.
16. The method for preparation of an ocular transparent tissue-visualizing suspension according to (14) or (15) above, wherein the solid preparation and/or the aqueous medium contains at least one compound selected from the group consisting of polyvinyl-based compounds and polyols.
17. An ophthalmic transparent tissue-visualizing preparation comprising a solid preparation comprising fine particles of a biodegradable macromolecular compound and an aqueous medium, separately and kept from contact with each other, wherein the aqueous medium and/or the solid preparation contains a salt of a trivalent metal and/or a salt of divalent metal so that a suspension obtained by mixing the solid preparation with the aqueous medium contains the salt of the trivalent metal at a concentration of not less than 0.01 w/v% and/or the salt of the divalent metal at a concentration of not less than 0.1 w/v%.
18. The ophthalmic transparent tissue-visualizing preparation according to (17) above, wherein the fine particles of a biodegradable macromolecular compound are those one g of which cannot completely be dissolved in less than 30 mL of water within 30 minutes at 20 °C.
19. The ophthalmic transparent tissue-visualizing preparation according to (17) or (18), wherein the solid preparation and/or the aqueous medium contains at least one compound selected from the group consisting of polyvinyl-based compounds and/or polyols.
20. The ophthalmic transparent tissue-visualizing preparation according to one of (17) to (19) above, wherein the content of the fine particles of a biodegradable macromolecular compound in the suspension is 0.005-10 w/v%.
21. The ophthalmic transparent tissue-visualizing preparation according to one of (17) to (20) above, wherein the solid preparation and the aqueous medium are contained in a single means for mixing and discharging them.
22. A method for enhancing visibility of transparent tissues of the eye comprising infusing the suspension according to one of (1) to (10) above into the eye to bring the suspension into contact with the transparent tissues of the eye.

### [Effect of the invention]

In surgical operations on transparent tissues of the eye, i.e., the vitreous body, the lens and the cornea, the present invention as defined above, when infused into the eye to be brought into contact with those transparent tissues of the eye for allowing the particles to adhere to them, i.e., the tissues which otherwise are hardly visible in the operative field, greatly enhances their visibility. Therefore, the present invention permits easier operation in such a surgery as well as surer and easier achievement of the purpose of the surgery. In particular, by infusing the ocular transparent tissue-visualizing suspension of the present invention into a vitreous cavity during intraocular surgery involving vitrectomy, such as diabetic retinopathy, retinal vein occlusion, macular edema, diabetic maculopathy, macular hole, epiretinal membrane formation, rhegmatogenous retinal detachment, and so on, countless numbers of its fine particles adhere to the vitreous gel, which, when irradiated with visible light, strongly scatter the light conforming the surface of the vitreous gel, and thereby remarkably enhance the visibility of the vitreous body, which was hardly visible before, thus allowing easier visual detection of it in the operative field through an operation microscope. Also in surgical operations which involve removal of the nucleus and cortex of the lens, the suspension, when infused into the lens capsule, likewise improves the visibility of them, for a countless numbers of its fine particles adhere to the nucleus and cortex of the lens and scatter light conforming the surface of them. Further, as the present invention employs fine particles which have no pharmacological activity, it will not induce unnecessary pharmacological reactions or side effects in the body. In particular, as the fine particles employed are biodegradable, even if part of them remain adhered to tissues of the eye after operation, they will be eliminated from the tissues of the eye with time through dissolution and/or decomposition, and excretion or absorption, thus being less likely to cause a problem.

### BRIEF DESCRIPTION OF DRAWINGS

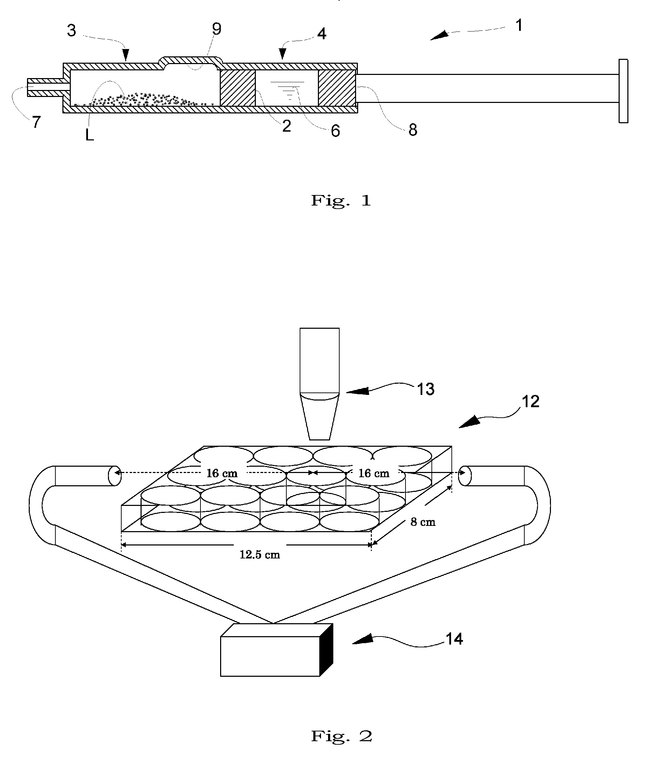

[FIG.1] A schematic cross sectional view of an example of ophthalmic transparent tissue-visualizing preparation in the form of a prefilled double-chamber syringe.
[FIG, 2] A schematic illustration of the method for illuminating and photographing a vitreous body sample in evaluation of visualization ability of tested suspensions.

### [Explanation of signs]

- 1 =: double chamber syringe
- 2 =: slidable partition
- 3 =: front chamber
- 4 =: rear chamber
- 7 =: discharge flow path
- 8 =: piston
- 9 =: bypassing flow path
- 12 =: 12-well plate
- 13 =: digital microscope
- 14 =: fiber light source

### BEST MODE FOR CARRYING OUT THE INVENTION

In the present invention, those "biodegradable macromolecular compound" may be employed which are pharmacologically inactive to the tissues of the eye of mammals, especially of a human, as well as to the body. There are no particular limitations as to the molecular weight of a macromolecular compound to be employed in the present invention, for it is sufficient that such a biodegradable macromolecular compound to be employed in the present invention is solid at an ordinary temperature and can be formed into fine particles. Its molecular weight usually is not less than 1500, preferably not less than 2000, more preferably not less than 3000, and most preferably not less than 5000, and usually not more than 200000, preferably not more than 100000, more preferably not more than 80000, and most preferably not more than 50000. However, those that fall outside these ranges may also be employed insofar as they serve to achieve the objective of the present invention.

Though various compounds may be employed as such biodegradable macromolecular compounds, it is preferable that the fine particles made thereof are of a property that one gram of them cannot be completely dissolved in less than 30 mL of water within 30 minutes at 20° C. It is because such solubility is sufficient for the fine particles to resist total dissolution and remain as light scattering sources during a usual course of a surgical operation. This degree of solubility corresponds to a solubility covering from "sparingly soluble", to "slightly soluble", "very slightly soluble" and "practically insoluble" as defined in the Japanese Pharmacopoeia, 14th edition. The Pharmacopoeia defines solubility based on whether one gram of a solute is dissolved in a solvent within 30 minutes at 20 ± 5 °C, with stirring for 30 seconds in every 5 minutes. In the present invention, this standard is substantially followed, and those fine particles are favorably employed that exhibit one of the following solubility levels when one gram of which is well stirred in water for 30 minutes at 20 °C.
(1) Sparingly soluble (i.e., complete dissolution cannot be achieved in less than 30 mL of water, but there exists a volume of water in the range of from not less than 30 mL and to less than 100 mL, in which complete dissolution can be achieved.)
(2) Slightly soluble (i.e., complete dissolution cannot be achieved in less than 100 mL of water, but there exists a volume of water in the range of from not less than 100 mL and to less than 1000 mL, in which complete dissolution can be achieved.)
(3) Very slightly soluble (i.e., complete dissolution cannot be achieved in less than 1000 mL of water, but there exists a volume of water in the range of from not less than 1000 mL and to less than 10000 mL, in which complete dissolution can be achieved.)
(4) Practically insoluble (i.e., at least 10000 ml of water is needed for complete dissolution).

There are no clear-cut limit as to the level of poorness in water solubility (length of time required for dissolution) of biodegradable macromolecular compounds that may be employed in the present invention. For example, such a level of solubility is sufficient that dissolution be achieved when stirred for one week in large excess of water at 37 °C, for even if some portion of the fine particles are left in the eye after a surgical operation, the amount of them will usually be very small and it is without problems insofar as the particles dissolve and lose their solid form even only gradually.

There is no clear-cut limits as to the mean size of the fine particles of a biodegradable macromolecular compound. However, considering easy handling in use and efficiency of scattering visible light, it in general is preferably about 0.01-500 µm, more preferably about 0.1-200 pm, still more preferably about 0.5-100 pm, and most preferably 1-60 µm.

Though there is a possibility that part of the fine particles of a macromolecular compound are left in the tissues after a surgical operation, no problem will be caused by this, as they are made of biodegradable macromolecular compound and eliminated with time from the eye, undergoing dissolution and degradation.

Various biodegradable macromolecular compounds may be employed in the present invention. Their examples include, but are not limited to, fatty acid polyesters such as polylactic acid (D-, L-, or DL-), polyglycolic acid, copoly(lactic acid/glycolic acid), polyhydroxybutyric acid, polyhydroxyvaleric acid, polycaprolactone, copoly(hydroxybutyric acid/glycolic acid), copoly(lactic acid/caprolactone), polyethylene succinate and polybutylene succinate and the like; polysaccharides and their derivatives such as starch and starch derivatives including soluble starch, pregelatinized starch and the like; cellulose and cellulose derivatives such as acetylcellulose, hydroxypropylmethylcellulose and the like, as well as chitosan, chitin, dextran and the like. Among these, most preferable are polylactic acid as well as polyglycolic acid and copoly(lactic acid/glycolic acid), which are of the property very similar to that of polylactic acid.

Any method may be employed as desired to make a biodegradable macromolecular compound into fine particles. Depending on the physicochemical properties of a macromolecular compound used, such methods may be employed as mechanical pulverization using, for example, a jet mill, and precipitation in a poor solvent utilizing the solubility difference of the macromolecular compound in various solvents (water, polar or nonpolar organic solvents).

In the present invention, examples of salts of trivalent metals include chloride or sulfate of aluminum, iron and the like, their double salts with a sulfate of a monovalent cation, hydroxides, lactates, and the like. More specifically, they include, but are not limited to, aluminum potassium sulfate, iron chloride (III), aluminum hydroxide, aluminum chloride, aluminum lactate, aluminum chloride hydroxide, or their hydrates. Among these, more preferred are aluminum potassium sulfate and iron chloride (III), and particularly preferred is aluminum potassium sulfate. The concentration of such a salt of a trivalent metal may be usually not less than 0.01 w/v%, preferably in the range of 0.01 w/v% to 1 w/v%, and most preferably 0.01 w/v% to 0.5 w/v%.

In the present invention, examples of salts of divalent metals include chloride, sulfate, carbonate, hydroxide, phosphate, citrate, acetate, bromide, lactate of magnesium, potassium, zinc, and the like. More specifically, they include, but are not limited to, magnesium chloride, calcium chloride, zinc chloride, magnesium sulfate, calcium hydroxide, magnesium hydroxide, magnesium carbonate, calcium carbonate, calcium phosphate, calcium citrate, magnesium citrate, calcium acetate, zinc acetate, calcium bromide, calcium lactate, and their hydrate. Among these, more preferred are magnesium chloride, calcium chloride, zinc chloride, and particularly preferred are magnesium chloride, and calcium chloride. The concentration of such a salt of a divalent metal may be usually not less than 0.1 w/v%, preferable in the range of 0.1 w/v% to 1 w/v%, and most preferably 0.1 w/v% to 0.5 w/v%.

The ocular transparent tissue-visualizing suspension according to the present invention may, along with the fine particles of a biodegradable macromolecular compound, contain a polyvinyl-based compound or a polyol, or both. A polyvinyl-based compound or a polyol favorably acts to promote dispersibility of the fine particles of a biodegradable macromolecular compound, so that, for example, they are instantly dispersed when mixed with an aqueous medium. Examples of particularly preferred polyvinyl-based compounds include, but are not limited to, polyvinylpyrrolidone and polyvinylalcohol, and examples of particularly preferred polyols include, but are not limited to, mannitol. Other polyvinyl-based compounds and polyols may also be employed as desired which are pharmacologically inactive and soluble in water. The amount of a polyvinyl-based compound or a polyol to be employed may usually be about 0.05-10 parts by weight per one part by weight of the fine particles of a macromolecular compound, though the amount may fall outside this range, for even a smaller amount of them has some effect corresponding to the amount, while higher amount of them leads to no disadvantage.

The ocular transparent tissue-visualizing suspension according to the present invention may be provided in the form of a suspension in which the fine particles of a biodegradable macromolecular compound are suspended in an aqueous medium.

An "aqueous medium", a component of the ocular transparent tissue-visualizing suspension according to the present invention, is a solution containing at least one salt selected from the group consisting of salts of trivalent metals and salts of divalent metals. When it contains a salt of a trivalent metal, the concentration of the salt is preferably not less than 0.01 w/v%, and, in general, preferably not more than 1 w/v%, though there is no particular upper limit. When it contains a salt of a divalent metal, the concentration of the salt is preferably not less than 0.1 w/v%, and, in general, preferably not more than 1 w/v%, though there is no particular upper limit. The aqueous medium may contain as desired isotonizers such as other salts or saccharides, buffering agents, and other additives which are acceptable to the ocular tissues, in particular, the intraocular tissues.

In the ocular transparent tissue-visualizing suspension according to the present invention, the content of the fine particles of a biodegradable macromolecular compound is preferably 0.005-10 w/v/% for the unfailing effect of visibility enhancement, and more preferably 0.01-5 w/v%, and most preferably 0.1-2 w/v%. However, the content may be outside this range, too, for a lower content than these, e.g., even 0.0001 w/v%, could achieve visualization, while a higher content than these is also applicable if it does not lead to inconvenience in handling, e.g., for letting the fine particles disperse. For convenience, the content usually may be set in the range of 0.01-5 w/v%. And, when one or more of the above-mentioned polyvinyl-based compounds or polyols is added further to increase dispersibility of the fine particles, the concentration of any of such compounds may be set as desired in the range of 0.1-5 w/v%. However, as a concentration below this range would still prove effective corresponding to the concentration, and a higher concentration would lead to no particular disadvantage, it is also allowed to employ such compounds at concentrations outside the range.

The ocular transparent tissue-visualizing suspension according to the present invention may be provided in a two-part type preparation which comprises, separately and keeping them from contacting with each other, a solid preparation such as a powder containing fine particles of a biodegradable macromolecular compound and an aqueous medium in which the powder is to be suspended to form a suspension when used in surgery. In this case, the preparation is so made that it contains either in its aqueous medium side or in its solid preparation side, or in both, most preferably in the aqueous medium side, one or more of the salts of trivalent metals and/or the salts of divalent metals, so that when the solid preparation and the aqueous medium are mixed, a suspension thus obtained contains at least one of the salts of trivalent metals or the salts of divalent metals, and that the concentration of the salt of the trivalent metal is not less than 0.01 w/v% and the content of the salt of the divalent metal is not less than 0.1 w/v%.

Furthermore, such a two-part type preparation may further contain one or both of the above-mentioned polyvinyl-based compounds and polyols, in its solid preparation side or in its aqueous medium side, or in both, and most preferably at least in the solid preparation side.

The above-mentioned two-part type preparation may be in such a form that the fine particles of a biodegradable macromolecular compound and the aqueous medium are enclosed in a single means for mixing and discharging.

In the above, the "means for mixing and discharging" may be in any form insofar as it allows to mix, according to manipulation from outside, the powder and the aqueous medium enclosed separately from each other, and then discharge, according to manipulation from outside, a mixture thus prepared to the outside. As such means, various double-chamber type syringes are well known (see Fig. 1). A double-chamber type syringe typically is of a cylindrical body at the front end of which is provided with a discharge flow path around which an injection needle can be (or already is) fitted, and through the other end of which is inserted a liquid-tight piston, and in an intermediate region of which is placed a liquid-tight partition that is inserted slidably in the longitudinal direction, thus forming two chambers, front and rear, within the cylindrical body. Forward of the partition is provided, by in the form of a recess defined in the interior surface of the cylinder, an elongated bypassing flow path which extends in the longitudinal direction over a range greater in length than the thickness of the partition. In the front chamber is usually enclosed a solid preparation, such as a dry powder, and in the rear chamber an aqueous medium (e.g., a buffer solution) to be combined with the former. The process of mixing and discharging is performed as follows. The piston inserted in the rear end is pushed to advance and, by a hydraulic pressure created by this, the slidable partition then is advanced forward within the syringe up to the central region of the bypassing flow path. The piston is advanced further until the aqueous medium contained in the rear chamber is forced to flow in the front chamber through the bypassing flow path, and the contents are thus mixed within the front chamber, and then the mixture is discharged through the discharge flow path by further advancement of the slidable partition which is caused by pressure applied on the piston. It is of particular advantage to provide the ocular transparent tissue-visualizing suspension of the present invention in a form in which the preparation is enclosed, separately and in a manner of keeping the components from contacting with each other, in such a single means for mixing and discharging, for such a form greatly enhances convenience in using the preparation during a surgical operation.

In the above, the solid preparation, such as a powder, which constitutes the two-part type preparation may be prepared, for example by once suspending the fine particles of a biodegradable macromolecular compound in a proper volume of an aqueous medium, such as a buffer solution, together with, as desired, one of the above-mentioned polyvinyl-based compound and salts of trivalent or divalent metals, and then subjecting the mixture to, e.g., lyophilization following quick freezing.

The ocular transparent tissue-visualizing suspension of the present invention may, as desired, contain: pharmaceutically acceptable additives, such as tonicity agents (salts like sodium chloride, potassium chloride, etc.; saccharides like glycerol, glucose, etc.; polyols like sorbitol, mannitol, propylene glycol, etc.; boric acid, borate, etc.), buffering agents (phosphate buffer, acetate buffer, borate buffer, carbonate buffer, citrate buffer, tris buffer, etc.), thickening agents (hydroxyethylcellulose, hydroxypropylcellulose, hydroxypropylmethylcellulose, carboxycellulose sodium, polyvinylalcohol, polyvinylpyrrolidone, polyethylene glycol, sodium alginate, etc.), stabilizers (sodium bisulfite, ascorbic acid, sodium ascorbate, dibutylhydroxytoluene, etc.), pH adjusting agents (hydrochloric acid, sodium hydroxide, phosphoric acid, acetic acid, etc.), and the like. Furthermore, a pharmacologically active ingredient may also be contained in the ocular transparent tissue-visualizing suspension of the present invention insofar as it does not conflict with the purpose of the present invention. The pH of the ocular transparent tissue-visualizing suspension of the present invention is generally adjusted to fall in the range of 4.0-8.0, preferably about 5.0-7.5.

The present invention will be described in further detail with reference to working examples, comparative examples, and test examples. However, it is not intended that the present invention be limited to those working examples.

### [Test Example 1] Visibility test on the vitreous body

### (Materials)

The following materials were employed for preparation of fine particles, their formulation into a suspension, and testing.
(1) D,L-polylactic acid (molecular weight: about 5000) (PLA0005, mftd. by Wako Pure Chemical Industries)
(2) Polyvinylpyrrolidone (Povidone K-30, mftd. by BASF Japan, Japanese Pharmacopoeia)
(3) D-mannitol (mftd. by Nakalai Tesque)
(4) Aluminum potassium sulfate dodecahydrate (mftd. by Wako Pure Chemical Industries)
(5) Iron chloride (III) hexahydrate (mftd. by Wako Pure Chemical Industries)
(6) Magnesium chloride (mftd. by Sigma)
(7) Calcium chloride dihydrate (mftd. by Wako Pure Chemical Industries)
(8) Zinc chloride (mftd. by Nakalai Tesque)
(9) Opeguard MA (mftd. by Senju Pharmaceutical Co., Ltd., intraocular-irrigating solution, containing (per 1 mL): glucose 1.5 mg, sodium chloride 6.6 mg, potassium chloride 0.36 mg, calcium chloride 0.18 mg, magnesium sulfate 0.3 mg, sodium bicarbonate 2.1 mg)

### (Preparation of fine particles)

An aqueous solution containing 1.11 % D-mannitol and 0.55 % Povidone K-30 was filtered through a 0.22 pm hydrophilic filter to make liquid A. Separately, a 10 % PLA0005 (acetone:ethanol = 4:6) solution was prepared, which then was filtered through a 0.22 pm hydrophobic filter to make liquid B.

Further, a suspension (liquid C) was prepared according to the following formula and procedure.

### <Liquid for suspension (liquid C)>

| | |
|---|---|
| Sodium chloride | 0.75 g |
| Potassium chloride | 0.16 g |
| Disodium hydrogen phosphate dodecahydrate | 0.25 g |
| Acetic acid | q.s. (pH 7.0) |
| Purified water | to 100 mL |

To purified water are added 0.75 g of sodium chloride, 0.16 g of potassium chloride, and 0.25 g of disodium hydrogen phosphate dodecahydrate to dissolve, and after pH is adjusted to 7.0 with acetic acid, purified water is added to make 100 mL.

The liquids A and B were mixed at a ratio of 9:1 in the following manner. Namely, to the liquid A, while stirred at 700-800 rpm on a stirrer, was added the liquid B at a rate of about 100 µL/sec to allow PLA0005 to precipitate as fine particles. The mixture was further stirred for about 30 minutes to make liquid D. After removing aggregation product with a sieve (mesh size 106 µm), lyophilization was performed in vials to obtain powder samples (fine particles) E. The procedure for this lyophilization was as follows. Namely, the liquid D was stored for 6 hours at -40° C to freeze, and after the pressure was reduced to 100 µmHg or lower at -40° C, allowed to dry for at least 24 hours. The temperature was raised from -40° C at a rate of 10° C until it reached +20° C. The drying process was further continued for at least 24 hours at +20° C and not higher than100 µmHg.

### (Preparation of test medium)

To purified water were added 0.75 g of sodium chloride, 0.16 g of potassium chloride, 0.25 g of disodium hydrogen phosphate to dissolve, and after pH was adjusted to 7.0 with acetic acid, the volume was adjusted 80 mL (liquid F). Separately, to the liquid F was added 1 g of magnesium chloride, aluminum potassium sulfate dodecahydrate, calcium chloride dihydrate, iron chloride (III) hexahydrate, or zinc chloride, and each of the solutions was made to volume to 100 mL with purified water to provide solutions each containing a salt of either a trivalent or divalent metal at a concentration of 1 w/v% (liquid G). Aliquots of the liquid G were taken and diluted with the liquid C so that the final concentration of a salt of a trivalent or divalent metal was at 0.005% to 0.75% to provide test media which contained a salt of a trivalent or divalent metal at different concentrations.

### (Evaluation of visualization effect on the vitreous body)

According to the following procedure, each suspension was examined for its ability of visualizing pig vitreous body. As a control, the liquid C was used instead of each test medium to prepare a control suspension.
1. The vitreous body is excised from a pig eye, and about 1 g of it is immersed in 5 mL of Opeguard MA in a beaker (hereinafter referred to as "liquid 1").
2. Fifty mg each of the powder sample E is suspended in 2 mL each of the test media (hereinafter referred to "test suspension"). The test suspension is injected into the solution 1 (hereinafter referred to as "liquid 2"), and then stirred to mix by hand.
3. The liquid is discarded from the liquid 2, leaving only the vitreous body behind.
4. Five mL of Opeguard MA is added to the beaker containing the vitreous body alone, and the beaker is stirred by hand.
5. Again, the liquid is discarded, leaving only the vitreous body behind.
6. The processes 4-5 are repeated one more time.
7. Five mL of Opeguard MA is added.
8. Only the vitreous body is transferred to a 12-well plate (12.5 cm x 8 cm, inner diameter: 22 mm, IWAKI) (see Fig. 2)
9. Using a digital microscope (model No. VHX-500, Keyence Corporation), following processes are carried out.
10. A fixed amount of light (fiber light source, Olympus GPS, Olympus Corporation) is irradiated horizontally from two opposing positions each of which is 16 cm away from a target well (see Fig. 2). The intensity of illumination on the target well is about 1000 Lux.
11. The magnification of the digital microscope is adjusted to 20x, at which most of the well is viewed in the field, and the focus is set on the surface of the vitreous body.
12. A still image is taken at the magnification of 20x from above the target well.
13. In the still image, the areas whose brightness is not less than 65 are designated.
14. Areas of not more than 30 pixel are eliminated to exclude small dots.
15. The total area selected in the processes 13-14 is determined.

The results are shown in Table 1.
[TABEL 1]

**Table 1.**

| Conc. of salt divalent or trivalent metal (w/v%) | Total area (mm²) | | | | | |
|---|---|---|---|---|---|---|
| | Aluminum potassium sulfate | Magnesium chloride | Iron chloride (III) | Calcium chloride | Zinc chloride | Control Conc. = 0) |
| 0.005 | 14.28 | 12.07 | - | - | - | 29.55 ± 9.08 |
| 0.01 | 62.15 | 10.35 | - | - | - | |
| 0.05 | 56.28 | 19.75 | - | - | - | |
| 0.1 | 43.75 | 89.8 | - | - | - | |
| 0.25 | 90.48 | 56.89 | 114.17 | 54.05 | 95.37 | |
| 0.5 | 133.01 | 104.71 | 90.56 | 108.04 | 95.85 | |
| 0.75 | 144.99 | 132.95 | 66.54 | 69.78 | 98.49 | |
| 1.0 | 128.56 | 122.58 | 74.18 | 115.93 | 94.83 | |

With control suspension, the total area which was selected was 29 ± 9.08 mm² (n=3). With a test suspension, aluminum potassium sulfate, a salt of a trivalent metal, at the concentration of 0.01 w/v%, the total area was 62.15 mm², indicating that it has a notably higher visualization ability compared with the control suspension. It showed very high visualization ability also at higher concentrations compared with that of the control suspension, and nearly maximum visualization ability was observed at concentrations around 0.5-1.0 w/v%. Iron chloride (III), a salt of a trivalent metal, also showed a markedly high visualization ability (measurement performed at and over 0.25 w/v% only). With magnesium chloride, a salt of a divalent metal, remarkably high visualization ability was shown compared with the control suspension at a concentration of 0.1 w/v% at which the total area of 89.8 mm², and it showed markedly high visualization ability also at higher concentrations compared with the control suspension, having nearly maximum visualization ability at concentration around 0.5-1.0 w/v%, too. Salts of other divalent metals, i.e., calcium chloride and zinc chloride, also showed markedly high visualization ability compared with the control suspension (measurement performed only at concentrations of 0.25 w/v% and over).

### [Preparation Example 1]

An ocular transparent tissue-visualizing suspension of the following formula is prepared by a conventional method using polylactic acid fine particles. The mean particle size of the polylactic acid fine particles is 20-30 µm.

| | |
|---|---|
| Polylactic acid fine particles | 1.0 g |
| Sodium chloride | 0.75 g |
| Potassium chloride | 0.16 g |
| Salt of a divalent or trivalent metal * | 0.1-1.0 g |
| Disodium hydrogen phosphate dodecahydrate | 0.25 g |
| Acetic acid | q.s. |
| Purified water | to 100 mL |
| pH | 7.0 |

| | |
|---|---|
| *) The salt of a divalent or trivalent metal was selected from aluminum potassium sulfate, magnesium chloride, calcium chloride dihydrate, iron chloride (III) and zinc chloride (also in the following examples). | |

### [Preparation Example 2]

An ocular transparent tissue-visualizing suspension of the following formula is prepared by a conventional method using fine particles of copoly(lactic acid/glycolic acid). The mean particle size of the copoly(lactic acid/glycolic acid) is 60-70 µm.

| | |
|---|---|
| Fine particles of copoly(lactic acid/glycolic acid) | 2 g |
| Sodium chloride | 0.75 g |
| Potassium chloride | 0.16 g |
| Salt of a divalent or trivalent metal | 0.1-1.0 g |
| Disodium hydrogen phosphate dodecahydrate | 0.25 g |
| Povidone K-30 | 1.0 g |
| D-mannitol | 1.0 g |
| Acetic acid | q.s. |
| Purified water | to 100 mL |
| pH | 7.0 |

### [Preparation Example 3]

An ocular transparent tissue-visualizing suspension of the following formula is prepared by a conventional method using polylactic acid fine particles.

| | |
|---|---|
| Polylactic acid | 5 g |
| Disodium hydrogen phosphate dodecahydrate | 0.1 g |
| Sodium chloride | 0.9 g |
| Sodium hydroxide | q.s. |
| Salt of a divalent or trivalent metal | 0.1-1.0 g |
| Sodium edetate | 0.1 g |
| Povidone K-30 | 5 g |
| D-mannitol | 5 g |
| Sterile purified water | to 100 mL |
| pH | 7.0 |

### [Preparation example 4]

An ocular transparent tissue-visualizing suspension of the following formula is prepared by a conventional method using fine particles of copoly(lactic acid/glycolic acid).

| | |
|---|---|
| Fine particles of copoly(lactic acid/glycolic acid) | 0.10 g |
| Sodium chloride | 0.55 g |
| Potassium chloride | 0.16 g |
| Dried sodium carbonate | 0.06 g |
| Sodium hydrogen phosphate | 0.18 g |
| Boric acid | 1.2 g |
| Borax | q.s. |
| Salt of a divalent or trivalent metal | 0.1-1.0 g |
| Povidone K-30 | 0.1 g |
| D-mannitol | 0.1 g |
| Sterile purified water | to 100 mL |
| pH | 7.3 |

### [Preparation Example 5]

An ocular transparent tissue-visualizing suspension of the following formula is prepared by a conventional method using polyglycolic acid fine particles.

| | |
|---|---|
| Polyglycolic acid fine particles | 1.0 g |
| Sodium chloride | 0.75 g |
| Potassium chloride | 0.16 g |
| Salt of a divalent or trivalent metal | 0.1-1.0 g |
| Disodium hydrogen phosphate dodecahydrate | 0.25 g |
| Povidone K-30 | 1.0 g |
| D-mannitol | 1.0 g |
| Acetic acid | q.s. |
| Purified water | to 100 mL |
| pH | 7.0 |

### [Preparation Example 6] Prefilled double-chamber syringe-type ocular transparent tissue-visualizing suspension

An aqueous solution containing 1.11 w/v% D-mannitol and 1.11 w/v% Povidone K-30 is filtered through a hydrophilic filter having a pore size of 0.22 pm to make Liquid F. Separately, a 10 w/v% PLA0005 (acetone:ethanol = 4:6) solution is filtered through a hydrophobic filter having a pore size of 0.22 pm to make liquid G. Phosphate buffers (pH7) containing 0.75% sodium chloride, 0.16% potassium chloride, 0.1-1.0 w/v% salt of a divalent or trivalent metal (selected from aluminum potassium sulfate, magnesium chloride, calcium chloride dihydrate, iron chloride (III), and zinc chloride) are prepared to make liquid H.
The liquid F and G are mixed at a ratio of 9:1 in the following manner. Namely, to the liquid F, stirred at 700-800 rpm on a stirrer, was added dropwise the liquid G at a rate of about 10-100 µL/sec to allow PLA0005 to precipitate in fine particles. This mixture is stirred for about 40 minutes (for 10 minutes of which, under reduced pressure), and aggregation products are removed with a sieve (mesh size 106 pm) to obtain a suspension J. This is dispensed, 2 ml each, in the front chamber 3 of double-chamber type syringes, whose schematic illustration is given in Fig 1, and is quick frozen and subjected to lyophilization to give a powder L. A prefilled double-chamber syringe-type ocular transparent tissue-visualizing-suspension is prepared by fitting a rubber plug, which serves as slidable partition 2 between the front chamber and the rear chamber 4, filling the rear chamber 4 with 2-mL of liquid H as an aqueous medium 6, and then fitting a plug which serves as piston 8. The numeral 9 indicates a longitudinal bypassing flow path, which is defined by a partial recess in the interior wall of the double chamber syringe 1. Lyophilization is carried out in the following manner. Namely, the suspension J is frozen by storing it at -40 °C for 6 hours, and after the pressure is reduced to 100 µmHg at -40° C, allowed to dry for at least 24 hours. The temperature then is allowed to rise from -40° C, at a rate of 10° C/hour, until it reaches +20° C. The drying process is further continued at +20 °C and at or below 100 µmHg for at least 24 hours.
The ocular transparent tissue-visualizing suspension of this preparation example is used in the following manner. Namely, the piston 8 is pushed in to advance, and, utilizing the pressure generated by this in liquid H, the medium enclosed on the rear chamber 4, the slidable partition 2 then is pushed to advance. When the rear edge of the slidable partition 2 reaches the bypassing flow path 9, the rear chamber 4 and the front chamber 3 are placed into communication with each other, and the liquid H starts to flow into the front chamber 3. By pushing in the piston 8 until it abuts on the slidable partition 2, all the liquid H is transferred to the front chamber 3, where it mixes with the powder L. After completion of the mixing, the piston 8 is further pushed in (together with the slidable partition 2) to advance, and the mixture liquid then is discharged out of the discharge flow path 7 (through a needle, etc. not shown) to the part on which a surgical operation is being performed.

### [Preparation Example 7]

An ocular transparent tissue-visualizing suspension is prepared of the following formula by a conventional method using polylactic acid fine particles.

| | |
|---|---|
| Polylactic acid fine particles | 1.0 g |
| Sodium chloride | 0.75 g |
| Potassium chloride | 0.16 g |
| Salt of a divalent or trivalent metal | 0.1-1.0 g |
| Disodium hydrogen phosphate | 0.25 g |
| Polyvinylalcohol | 1.0 g |
| Acetic acid | q.s. |
| Purified water | to 100 mL |
| pH | 7.0 |

### [Industrial Applicability]

The present invention as defined above greatly enhances visibility of transparent tissues in surgical operations on those tissues of the eye, i.e., the vitreous body, the lens, and the cornea, which otherwise are hardly visible in the operative field, without causing unnecessary reactions or side effects in the body, thus permitting easier operations as well as surer achievement of the purpose of the surgical operations with less difficulty.

## Claims

1. An ocular transparent tissue-visualizing suspension comprising, in an aqueous medium, fine particles of a biodegradable macromolecular compound and at least one salt selected from the group consisting of salts of divalent metals and salts of trivalent metals, wherein the composition is designed to be infused into the eye to be brought into contact with transparent tissues of the eye to enhance the visibility thereof.

2. The ocular transparent tissue-visualizing suspension according to claim 1, wherein the fine particles of a biodegradable macromolecular compound are those one g of which cannot completely be dissolved in less than 30 mL of water within 30 minutes at 20 °C.

3. The ocular transparent tissue-visualizing suspension according to claim 1 or 2, wherein the biodegradable macromolecular compound is selected from the group consisting of polylactic acid, polyglycolic acid, and copoly(lactic acid/glycolic acid).

4. The ocular transparent tissue-visualizing suspension according to one of claims 1 to 3, wherein the concentration of the salt of a trivalent metal is 0.01-1 w/v% and/or the concentration of the salt of a divalent metal is 0.1-1 w/v%.

5. The ocular transparent tissue-visualizing suspension according to one of claims 1 to 4, wherein the salt of a trivalent metal is selected from aluminum salts, and iron salts, and wherein the divalent salt is selected from magnesium salts, calcium salts, and zinc salts.

6. The ocular transparent tissue-visualizing suspension according to one of claims 1 to 5, wherein the salt of a trivalent metal is aluminum potassium sulfate.

7. The ocular transparent tissue-visualizing suspension according to one of claims 1 to 6, wherein the salt of a divalent metal is magnesium chloride or calcium chloride.

8. The ocular transparent tissue-visualizing suspension according to one of claims 1 to 7, further comprising a polyvinyl-based compound, which may be polyvinylpyrrolidone or polyvinylalcohol, and/or a polyol.

9. The ocular transparent tissue-visualizing suspension according to claim 8, wherein the polyvinyl-based compound is polyvinylpyrrolidone and the polyol is mannitol.

10. The ocular transparent tissue-visualizing suspension according to one of claims 1 to 9, wherein content of the fine particles of the biodegradable macromolecular compound is 0.005-10 w/v% .

11. A method for preparation of an ocular transparent tissue-visualizing suspension comprising the steps of
providing a solid preparation comprising fine particles of a biodegradable macromolecular compound,
providing an aqueous solution containing at least one salt selected from the group consisting of salts of trivalent metals and salts of divalent metals, wherein the concentration of the salt of a trivalent metal is not less than 0.01 w/v%, and/or wherein the concentration of the salt of a divalent metal is not less than 0.1 w/v%, and
suspending the fine particles in the aqueous solution.

12. The method for preparation of an ocular transparent tissue-visualizing suspension according to claim 11, wherein the fine particles of a biodegradable macromolecular compound are those one g of which cannot completely be dissolved in less than 30 mL of water within 30 minutes at 20 °C.

13. The method for preparation of an ocular transparent tissue-visualizing suspension according to claim 11 or 12, wherein the solid preparation and/or the aqueous solution further comprises at least one compound selected from the group consisting of polyvinyl-based compounds and polyols.

14. A method for preparation of an ocular transparent tissue-visualizing suspension comprising the steps of
providing a solid preparation comprising fine particles of a biodegradable macromolecular compound and at least one salt selected from the group consisting of salts of trivalent metals and salts of divalent metals, and
forming a suspension by mixing the solid preparation with an aqueous medium so that the concentration of the salt of a trivalent metal is not less than 0.01 w/v% and/or the concentration of the salt of a divalent metal is not less than 0.1 w/v%.

15. The method for preparation of an ocular transparent tissue-visualizing suspension according to claim 14, wherein the fine particles of a biodegradable macromolecular compound are those one g of which cannot completely be dissolved in less than 30 mL of water within 30 minutes at 20 °C.

16. The method for preparation of an ocular transparent tissue-visualizing suspension according to claim 14 or 15, wherein the solid preparation and/or the aqueous medium contains at least one compound selected from the group consisting of polyvinyl-based compounds and polyols.

17. An ophthalmic transparent tissue-visualizing preparation comprising a solid preparation comprising fine particles of a biodegradable macromolecular compound and an aqueous medium, separately and kept from contact with each other, wherein the aqueous medium and/or the solid preparation contains a salt of a trivalent metal and/or a salt of divalent metal so that a suspension obtained by mixing the solid preparation with the aqueous medium contains the salt of the trivalent metal at a concentration of not less than 0.01 w/v% and/or the salt of the divalent metal at a concentration of not less than 0.1 w/v%.

18. The ophthalmic transparent tissue-visualizing preparation according to claim 17, wherein the fine particles of a biodegradable macromolecular compound are those one g of which cannot completely be dissolved in less than 30 mL of water within 30 minutes at 20 °C.

19. The ophthalmic transparent tissue-visualizing preparation according to claim 17 or 18, wherein the solid preparation and/or the aqueous medium contains at least one compound selected from the group consisting of polyvinyl-based compounds and/or polyols.

20. The ophthalmic transparent tissue-visualizing preparation according to one of claims 17 to 19, wherein the content of the fine particles of a biodegradable macromolecular compound in the suspension is 0.005-10 w/v%.

21. The ophthalmic transparent tissue-visualizing preparation according to one of claims 17 to 20, wherein the solid preparation and the aqueous medium are contained in a single means for mixing and discharging them.

22. A method for enhancing visibility of transparent tissues of the eye comprising infusing the suspension according to one of claims 1 to 10 into the eye to bring the suspension into contact with the transparent tissues of the eye.
